# EUROPEAN PATENT APPLICATION

(11) **EP 3 912 621 A1**
(43) Date of publication of application: **24.11.2021**
(21) Application number: 20175522.0
(22) Date of filing: 19.05.2020
(51) Int. Cl.: A61K 31/05, A61P 23/00, A61P 25/00, A61P 25/06

(54) **PROPOFOL FOR DIAGNOSIS OF PRIMARY HEADACHE**

(71) Applicant: tesa Labtec GmbH, 40764 Langenfeld (DE)
(72) Inventor: Lubenow, Helge, 40764 Langenfeld (DE); Niese, Svenja, 40597 Düsseldorf (DE); Ernst, Steffen, 435 37 Mölnlycke (SE)
(74) Representative: Hemsath, Lars

(57) **Abstract**

The present invention relates to a method of diagnosis of primary headache. More particular, the invention relates to a method of differentiating between primary and secondary headache in a human subject by applying a biomarker for primary headache. In a particular aspect, the invention relates to a method for the diagnosis of migraine. In one particular aspect the invention relates to the field of theranostic and personalized medicine.

## Description

### FIELD OF THE INVENTION

The present invention relates to a method of diagnosis of primary headache. More particular, the invention relates to a method of differentiating between primary and secondary headache in a human subject by applying a biomarker for primary headache. In a particular aspect, the invention relates to a method for the diagnosis of migraine. In one particular aspect the invention relates to the field of theranostic and personalized medicine.

### BACKGROUND OF THE INVENTION

Headaches are a worldwide prevalent disorder that can be found in many settings, which are also common complaints in the emergency room. Headaches can result from many conditions. Since not all headache conditions are best managed in a similar fashion, therapeutic management varies strongly depending on the specific type of headache, accurate diagnosis is an important factor in therapeutic outcome.

Together with dental caries, migraine and tension-type headaches are the 3 most common diseases in the world, with migraine being ranked the third-highest cause of disability worldwide in both males and females under the age of 50 years (Cephalalgia. 2018 JaN; 38(1); 1-211).

One classification system for headache is the International Classification of Headache Disorders (ICHD) published by the International Headache Society (IHS). Headaches are broadly classified as "primary", "secondary" and "cranial neuropathies, facial pains and other headaches" as third category. More than 90% of headaches are primary. Primary headaches differ from secondary in that a primary headache is due to the actual headache condition itself and cannot be ascribed to another cause, whereas a secondary headache is a symptom of a further underlying condition.

The category "cranial neuropathies, facial pains and other headaches" describes a number of disorders, which can be attributed to dysfunction or afferent activation of isolated craniofacial nerves, rather than true headaches. Headaches of this category are generally characterized by neuropathic-type pain in a specific cranial nerve distribution, i.e. a pain is perceived in a certain innervated area of the head or face. In consequence, a headache of the category "cranial neuropathies, facial pains and other headaches" is classified on the basis of its distinct clinical characteristic and etiology.

In clinical practise, the diagnosis of primary versus other types of headaches, in particular secondary headaches, poses significant challenges. This applies especially to emergency room setting, where a patient may present with an acute headache. Several secondary headaches require acute and dedicated therapeutic intervention to prevent further harm and lasting damage. An incorrect diagnosis of a secondary headache as a primary headache can results in severe, potentially fatal consequences for the patient.

In consequence, patients presenting with an acute headache are often subjected to a series of diagnostic means to examine for a secondary headache, including imaging methods such as computer tomography (CT), magnetic resonance imaging, magnetic resonance tomography (MRT) or functional methods including electroencephalography (EEG). The diagnosis of primary headache requires a complex, consensus-based classification scheme. One such classification scheme is the International Classification of Headache Disorders 3rd (ICHD-3) published by the Headache Classification Committee of the International Headache Society (IHS) (Cephalalgia. 2018 Jan; 38 (1):1-211).

In practice, a primary headache often is diagnosed only subsequently to the exclusion of a secondary headache. Prior to this laborious time-consuming diagnosis, the patient cannot receive any treatment, meaning that he continues suffering from headache, because a symptomatic treatment would interfere with the diagnosis of the underlying medical cause. Furthermore, the present standard of diagnosis involves significant costs to the medical system. Therefore, the entire procedure is not only stressful for the patient but is also economically disadvantegous.

Hence, it is an objective of the present invention to provide an improved diagnostic means that enables the diagnosis of a primary headache, in particular without the need of a prior exclusion of a secondary headache. It is furthermore an objective of the present invention to provide a biomarker for primary headache, which allows a convenient and effective means to differentiate between a primary and a secondary headache in a human subject.

### SUMMARY OF THE INVENTION

These objectivev are solved with propofol for use in the diagnosis of a primary headache in a human subject.

The inventors have found that the administration of propofol to a subject suffering from headache induces a response in patients with primary headaches, while propofol has no such effect in patients with secondary headaches or cranial neuropathies, facial pains and other headaches and that therewith a dagnostic of primary headache is possible. Hence, the responsiveness of a patient with headache to propofol can be used as a biomarker for the diagnosis of a primary headache. Furthermore, it allows a fast and easy way of differentiating between a primary and a secondary headache. Therewith the diagnosis of primary headache is improved in particular in terms of patient convenience and socioeconomic considerations. Moreover, in a particular aspect propofol according to the invention is applied in the field of personalized medicine and theranostics; in particular to allow for more optimized therapeutic regimes individualized for a respective patient.

The active principle for use in the diagnosis according to the invention is propofol (C₁₂H₁₈O). Propofol is the INN for 2,6-diisopropylphenol (CAS number 2078-54-8). It belongs to the group of anesthetics and is classified as a sedative hypnotic. Propofol is known to reduce tension and anxiety and to induce calmness (sedative effect) and/or sleep (hypnotic effect). Hence propofol has a sedative and/or hypnotic effect when administered in an anesthetic dose. It is typically applied intravenously and then requires vital sign monitoring and life support readiness. Propofol is commercially available for example under the tradenames Disoprivan®, Diprivan® or Recofol®. It is assumed to work at least partially via a receptor for gamma-aminobutyric acid (GABA).

According to the invention a response of a patient suffering from headache to propofol is assessed preferably within a particular time frame after the administration of propofol. This improves attributing the response of the patient clearly to propofol. The asssement of the response can be performed within 240 minutes after the administration of the substance. Most preferred the assessment is accomplished within 120 min, most preferred 120 min after the administration of propofol. In certain ambodiments the assessment can be accomplished even earlier, e.g. within 90 minutes, 60 minutes or 30 minutes after the administration.

The dose to be administered for the diagnostic purposes can differ from the known therapeutic doses of propofol, in particular it can exceed the dose for its therapeutic sedative and/or hypnotic effect, but still remains below a dose with an anaesthetic effect. In a preferred embodiment of the invention propofol is administered via a pharmaceutical film, in particular a buccal (mucoadhesive) film.

In a particular preferred embodiment of the invention, propofol is used in the diagnosis of a migraine in a human subject.

After the patients having been diagnosed according to the invention they can be subjected to the treatment of primary headache. Various treatment options or regimes for primary headache and/or migraine are known to the skilled person. In a particularly preferred embodiment the primary headache subsequently is treated with propofol.

### DETAILED DESCRIPTION OF THE INVENTION

The term "headache" as used in the context of this invention refers to a pain, a feeling of discomfort and/or disorientation in the face, head, or neck. Headaches can vary in symptoms including in terms of location, intensity and how frequently they occur. Due to the variation in types of headaches, different categorization systems have been created, such as the International Classification of Headache Disorders (ICHD) ICH by the International Heachache Society (IHS).

According to this classification system, headaches can be broadly classified as as "primary", "secondary" or as third category "cranial neuropathies, facial pains and other headaches" (Cephalalgia. 2018 Jan;38(1):1-211). In a primary headache the headache is the main medical problem itself, even though associated factors such as muscle tension or exposure to certain foods, may be identified. Examples of primary headaches include migraine, tension headache or cluster headache.

In a secondary headache, the headache is the symptom of a further underlying condition. An example of a secondary headache is a headache due to neck injuriy or sinus infection. Further examples of secondary headaches include headaches from infection, inflammation, head injury, trauma, stroke, vascular disorders, brain bleeding or tumors.

Examples of cranial neuropathies, facial pain and other headaches include pain attributed to a lesion or disease of the trigeminal nerve and pain attributed to a lesion or disease of the glossopharyngeal nerve.

According to the invention, a response of a subject to propofol indicates a primary headache. Therewith it allows the differentiation of a primary headache from a secondary headache. The responsiveness of the subject to propofil according to the invention is assessed by the mitigation of at least one symptom of primary headache post administration of propofol. In the context of the invention the mitigation of at least on symptom of a headache is denominated as a "response".

The symptom of primary headache can be selected from the group consisting of pain, cranial autonomic symptoms, symptoms of cutaneous allodynia, pericranial tenderness on manual palpation, headache, nausea, vomiting, photophobia, phonophobia, aura symptom, brainstem symptom, retinal symptom, anorexia, pallor, nystagmus, ataxia, fearfulness, irritability, malaise, conjunctival injection, lacrimation, nasal congestion, rhinorrhea, eyelid edema, forehead sweating, facial sweating, miosis, ptosis, restlessness and/or agitation or combinations thereof. A response to at least one of these symptoms after administration of propofol indicates a primary headache.

According to a preferred embodiment of the invention the symtoms to be assessed in order to identify a patient's response to propofol are the disappearance of severe headache, as well as all or a selection of vestibular, visual or auditory symtoms, in particular of nausea, migraine aura, photophobia and auditory disturbances.

These symptoms can be further specified. For example, a more specific embodiment of the symptom "aura symptom" is a reversible aura symptom, a prolonged aura symptom or an acute-onset aura symptoms. In yet another embodiment the response is in an aura symptom selected from reversible aura symptoms comprising visual, sensory, speech, language, motor, brainstem and/or retinal aura symptoms. In a different embodiment of the invention, the response is in a brainstem symptom selected from dysarthria, vertigo, tinnitus, hypacusis, diplopia, ataxia not attributable to sensory deficit and/or decreased level of consciousness. In yet another embodiment, the response is in a retinal symptom selected from reversible, monocular, positive and/or negative visual phenomena comprising clinical visual field examination and/or subject's drawing of a monocular field defect. In a different embodiment, the response is in pain assessed on a standardized scale known to the skilled person.

The term "pain" in the context of the present invention refers to a distressing feeling arising from a sensory or emotional experience. Pain is a complex sensory sensation triggered by nociceptors of the peripheral nervous system. The perception of pain is processed and interpreted in the central nervous system (CNS). There are close interactions between the perception of pain and the mental state of the subject. Pain can be caused by an intense or harmful stimulus. Pain can refer to a symptom of a disease such as primary headache, migraine and/or be a disease itself. Pain may be perceived over an extended period of time, whereby the underlying cause cannot or only hardly be treated, or cannot be identified at all.

According to the invention, response to propofol can be assessed also as a mitigation of pain. Various ways are known to the skilled person for the assessment of pain, such as Alder Hey Triage Pain Score, Behavioral Pain Scale (BPS), Brief Pain Inventory (BPI), checklist of nonverbal pain indicators (CNPI), Clinical Global Impression (CGI), COMFORT scale, Color Scale for Pain, Critical-Care Pain Observation Tool (CPOT), Dallas Pain Questionnaire, Descriptor differential scale (DDS), Dolorimeter Pain Index (DPI), Edmonton Symptom Assessment System, Face Legs Activity Cry Consolability scale, Faces Pain Scale - Revised (FPS-R), Global Pain Scale, Mankoski Pain Scale, McGill Pain Questionnaire (MPQ), Multiple Pain Rating Scales, Neck Pain and Disability Scale -NPAD, Numerical 11 point box (BS-11), Numeric Rating Scale (NRS-11), Oswestry Disability Index, Palliative Care Outcome Scale (PCOS), Roland-Morris Back Pain Questionnaire, Support Team Assessment Schedule (STAS), Wharton Impairment and Pain Scale (WIPS), Wong-Baker FACES Pain Rating Scale, Visual analog scale (VAS), Pediatric Pain Questionnaire (PPQ) or Premature Infant Pain Profile (PIPP).

Pain can also be classified according to its duration into acute and chronic pain. Therefore, the use of propofol in a method of diagnosis according to the invention may involve a response in acute and/or chronic pain. Pain can also be classified according to its quality and intensiveness. Affective pain quality refers to the subjective perception of the pain, such as violent, paralyzing, agonizing, martyring or terrible. Sensory pain perception refers to the actual perception of the pain for example as pulling, dull, drilling, stabbing, stinging, pressing, burning or knocking. According to the invention the response of the human subject can also either be in the affective pain quality and/or sensory pain quality.

Moreover, pain can be classified into mild, moderate and strong pain. For example, in the NRS-11, a rating of 0 refers to no pain, a rating of 1 - 3 refers to mild pain, a rating of 4 - 6 refers to moderate pain and a rating of 7 - 10 in this scale refers to strong or severe pain. In the NRS-11 scale, a reduction of pain would correspond to a reduction of the numerical value. Therefore, the diagnosis of a primary headache according to the invention can be based on a change of the claissification of the pain, e.g. before the administration of propofol the pain qualified as moderate and afterwards is changed to the mild.

According to the invention propofol can also be used for the diagnosis of a certain type of primary headache. The type of headache can be selected from the group consisiting of migraine, tension-type headache, trigeminal autonomic cephalalgias or other primary headache disorders including primary cough headache, primary exercise headache, primary headache associated with sexual activity, primary thunderclap headache, cold-stimulus headache, external-pressure headache, primary stabbing headache, nummular headache, hypnic headache or new daily persistent headache (NDPH). In a most preferred embodiment a migraine is diagnosed.

Migraine is the most common disabling primary headache disorder. Migraine has two major types, migraine without aura and migraine with aura. Migraine without aura is a clinical syndrome characterized by headache with specific features and associated symptoms, whereas migraine with aura is primarily characterized by the transient focal neurological symptoms that usually precede a headache. Symptoms in migraine without aura include fatigue, difficultly in concentrating, neck stiffness, sensitivity to light and/or sound, nausea, blurred vision, yawning and pallor.

In order to diagnose a migraine, preferably, a response in at least one symptom selected from the group consisting of aura symptoms, brainstem symptoms, retinal symptoms can be assessed (outlined in detail above). More specifically, preferred migraine symptoms are e.g. aura, nausea, photophobia, hemiparesis and phonophobia. It can be advantageous to combine this approach with the assessment of at least one symptom, which is not related to migraine ("non-migraine symptoms"). Therefore, in a preferred embodiment of the invention the diagnosis of a migraine includes the assessment of the responsiveness of the subject to at least one migraine specific symptom and to at least one non-migraine symptom.

Symptoms which are specific for migraine are generally known to the skilled person, e.g. from classification such as the ICHD-3 (Cephalalgia. 2018 Jan;38(1):1-211 or the guidelines from the European Headache Federation or the Refractory Headache Special Interest Section (RHSIS) of the American Headache Society (AHS).

As outlined above, it is advantageous that the assessment of the response of the subject is accomplished within a certain time frame after the administration of propofol. The asssement of the response preferably is performed within 240 minutes after the administration of the substance. Most preferred the assessment is accomplished within 120 min, most preferred 120 min after the administration of propofol. The assessment 120 min after the administration of propofol is particularly preferred in the diagnosis of migraine. However, in particular in emergency settings or when the patients suffer from severe pain an early assessment can be of great importance, in order to start the subsequent treatment as soon as possible. Thus it can also be preferred that the assessment is accomplished within 60 or 30 min after the administration of propofol.

The method according to the invention requires an effective dose of propofol. The dose is not necessarily identical to the therapeutically effective dose when propofol is used to induce or maintain a hypnotic or sedative effect. In order to obtain a fast response of the patient it may even be advantageous to administer a dose which exeeds the therepeutically effective hypnotic or sedative dose. Preferably propofol is used as a single administration.

Propofol for use in a method according to the invention can be given as a fixed dose or a body weight adjusted dose. As a fixed dose, preferably 10 to 20 mg are administered. If a body weigh adjusted does is applied, the preferred dose is 0.15 to 0.28 mg/kg.

The route of administration in the method according to the invention is not specifically limited. Any route is possible, comprising parenteral, oral, nasal, ocular, aural, transmucosal or transdermal administration, e.g. via an oral film. Most preferred is the parenteral or transmucosal single administration of propofol. A preferred dosage form is a buccal film, in particular a mucoadhesive oral dissolvable film.

After diagnosis of a primary headache and/or a migraine according to the invention, the subject can be treated with approaches and regimens known to the skilled person. Possible active ingredients for the treatment of primary headache are e.g. triptans, ditans such as lasmiditan, NSAIDs, glucocorticoid steroids, salicylates and derivatives thereof, phenylacetic acid and derivatives thereof, 2-phenylpropionic acid and derivatives thereof, 4-aminophenol and derivatives thereof, pyrazolones, selective COX2 inhibitors, anti-depressants, anticonvulsant drugs, opioids, muscle relaxants, barbiturates, benzodiazepines, etomidates, ketamine, propofol, anti-histamines and/or local anesthetics. Most preferred is the subsequent treatment with propofol.

In a certain embodiment of the invention propofol can be administred to the subject to be diagnosed by administration of a buccal (mucoadhesive) film. **The** formulation of the dosage unit can comprise a solvent, a polymer and propofol. Optionally the formulation of the dosage unit according to the invention can comprise a plasticizier. Other additives may be added to blend for the various purposes and include, but are not limited to bulk fillers, binding agents, thickening agents, softeners, surfactants, stabilizing agents, buffering agents, emulsifiers, disintegrants, flavoring agents, sweetening agents, colorants and the like, which may provide a benefit, but are not essential for forming the dosage unit matrix or for the pharmaceutical activity of the dosage unit.

In a preferred embodiment, the composition for producing the film comprises:
(a) solvent up to 95% by weight, up to 90% by weight, up to 80% by weight, up to 70% by, or up to 60% by weight; and
(b) polymer up to 35 % by weight, up to 30% by weight, up to 25%, by weight, or up to 20% by weight; and
(c) popofol up to 30% by weight, up to 20% by weight, up to 10% by weight, or up to 5% by weight; and
(d) optionally a plasticizer of up to 15 % by weight, up to 10% by weight or up to 5 % by weight;
whereby preferably the polymer is a hydrophilic polymer.

In a preferred embodiment, the composition for producing the film comprises:
a. solvent: 50 - 95 % by weight; preferably 60 - 85% by weight, more preferably, 60 - 75% by weight; and
b. polymer: 1 - 35% by weight, preferably 5 - 30% by weight, more preferably, 10 - 25% by weight, and
c. popofol: 0.1 - 30% by weight, preferably 1 - 20% by weight, more preferably 1 - 10 % by weight; and
d. optionally plasticizer: 0.1 - 15 % by weight, preferably 1 - 10 % by weight, more preferably 2 - 6% by weight;
whereby preferably the polymer is a hydrophilic polymer.

In a further preferred embodiment, the solid content of the composition for producing the film comprises
a. polymer at least 60%, preferably at least 70% and more preferably at least 80 % (w/w) of the solid content; and
b. plasticizer up to 30 %, preferably up to 25% and more preferably up to 20 % (w/w) of the solid content; and
c. propofol up to 25%, preferably 22.5 % and more preferably up to 20 % (w/w) of the solid content
whereby preferably the polymer is a hydrophilic polymer.

Preferably, the film preferably is dissolvable in water. Then, when applied onto the mucosa, the film dissolves. Upon such dissolution of the film propofol more easily penetrates and permeates through the mucosa.

The dosage unit of this embodiment is particularly suitable for transmucosal systemic delivery of propofol.

The film preferably is formed by polymers, in particular by hydrophilic polymers. Polymers can affect the mucoadhesivness of the dosage unit, for example the type of polymer selected or the ratio of polymer in the formulation can affect the mucoadhesiveness of the dosage unit. The film in the pharmaceutical preparation of the invention thus preferably comprises at least one hydrophilic polymer. In a particularly preferred embodiment, all polymers of the film are hydrophilic polymers. A polymer can be selected from the group of chitosan and derivatives to modify the time of mucoadhesion. A polymer can be selected from the group of chitosan, PVP, polyacrylic acid and/or poloxamer to increase the residence time by increasing mucoadhesion. Advantageously, the residence time can be modified by the selection of a polymer. Preferentially chitosan is selected as polymer to increase the mucoadhesiveness and therefore the time of residence of the dosage unit.

The formulation of the dosage unit can comprise up to 15 % by weight polymer, preferably between 1 to 10 % by weight polymer.

In a preferred embodiment of the invention the formulation of the film consists of at least 60 % (w/w) hydrophilic polymer, even more preferred 70% (w/w) hydrophilic polymer and most preferred at least 80% (w/w) hydrophilic polymer.

### Preferred embodiments of the invention and studies for its clinical evaluation

Clinical validation for use of a 10-20mg propofol buccal film (mucoadhesive Oral Dissolvable Film (mOFD)) as diagnostic aid in the differentiation between primary and secondary headaches and the diagnosis of migraine.

### 1. Emergency room setting

Multi-center, double-blind study in patients presenting with severe headache in hospital emergency rooms without other symptoms allowing for the differentiation between primary and secondary headaches. Following informed consent 50 male and female (approx.. 30% male) adult patients (18-65 years) receive a single dose of 10-20mg propofol mODF as first step in the otherwise standard diagnostic process. Patients will be assessed for 30, 60, 90 and 120min post dose headache pain relief. The primary endpoint is 120 min post dose pain relief. All patients are subject to the standard diagnostic measures. Results are compared with the diagnostic outcome (primary versus secondary) headaches by conventional methods. The diagnostic concept of the use of a 10-20mg propofol ODF is considered valid if ≥70% primary headaches are correctly identified with less than 5% false negative secondary headache forms (p=0.05).

### 2. Migraine diagnostic.

Randomized, multi-center, placebo-controlled, double-blind, cross-over study in patients presenting with a headache attack to a migraine center in the course of focused migraine diagnosis. Following informed consent 100 male and female (approx. 30% male) adult patients (18-65 years) receive a single dose of 10-20mg propofol mODF per ongoing headache episode. Patients will be assessed at regular intervals up to 240 mins post dose for all headache and non-headache symptoms. Each patient will receive one verum and one placebo product.

The diagnostic concept of the use of propofol according to the invention, in particular of 10-20mg propofol mODF, is considered valid if verum leads to a statistically significant improvement of headache pain and at least 1 other typical migraine symptom like nausea or photophobia or auditory disturbances in comparison to placebo.

## Claims

1. Propofol for use in a method of diagnosis of a primary headache in a human subject.

2. Propofol for use in a method of diagnosis of a primary headache in a human subject according to claim 1 **characterized in that** the primary headache is differentiated from a secondary headache.

3. Propofol for use in a method according to claims 1 or 2 **characterized in that** the responsiveness of the subject to the administration of propofol is used as a marker that the subject suffers from primary headache.

4. Propofol for use in a method according to one or more of claim 1 to 3 **characterized in that** a response to propofol is assessed in the mitigation of at least one symptom selected from the group consisiting of pain, cranial autonomic symptoms, symptoms of cutaneous allodynia, pericranial tenderness on manual palpation, headache, nausea, vomiting, photophobia, phonophobia, aura symptom, brainstem symptom, retinal symptom, anorexia, pallor, nystagmus, ataxia, fearfulness, irritability, malaise, conjunctival injection, lacrimation, nasal congestion, rhinorrhea, eyelid edema, forehead sweating, facial sweating, miosis, ptosis, restlessness and/or agitation or combinations thereof.

5. Propofol for use in a method according to claim 4 **characterized in that**
a. the aura symptom is selected from reversible aura symptoms comprising visual, sensory, speech, language, motor, brainstem and/or retinal aura symptoms; and/or
b. the brainstem symptom is selected from dysarthria, vertigo, tinnitus, hypacusis, diplopia, ataxia not attributable to sensory deficit and/or decreased level of consciousness; and/or
c. the retinal symptom is selected from reversible, monocular, positive and/or negative visual phenomena comprising clinical visual field examination and/or subject's drawing of a monocular field defect; and/or
d. pain is assessed on a standardized pain scale, preferably selected from Alder Hey Triage Pain Score, Behavioral Pain Scale (BPS), Brief Pain Inventory (BPI), checklist of nonverbal pain indicators (CNPI), Clinical Global Impression (CGI), COMFORT scale, Color Scale for Pain, Critical-Care Pain Observation Tool (CPOT), Dallas Pain Questionnaire, Descriptor differential scale (DDS), Dolorimeter Pain Index (DPI), Edmonton Symptom Assessment System, Face Legs Activity Cry Consolability scale, Faces Pain Scale - Revised (FPS-R), Global Pain Scale, Mankoski Pain Scale, McGill Pain Questionnaire (MPQ), Multiple Pain Rating Scales, Neck Pain and Disability Scale -NPAD, Numerical 11 point box (BS-11), Numeric Rating Scale (NRS-11), Oswestry Disability Index, Palliative Care Outcome Scale (PCOS), Roland-Morris Back Pain Questionnaire, Support Team Assessment Schedule (STAS), Wharton Impairment and Pain Scale (WIPS), Wong-Baker FACES Pain Rating Scale, Visual analog scale (VAS), Pediatric Pain Questionnaire (PPQ) or Premature Infant Pain Profile (PIPP).

6. Propofol for use in a method according to any or more of claims 1 to 5 **characterized in that** the primary headache is selected from the group consisting of migraine, tension-type headache, trigeminal autonomic cephalalgias or other primary headache disorders including primary cough headache, primary exercise headache, primary headache associated with sexual activity, primary thunderclap headache, cold-stimulus headache, external-pressure headache, primary stabbing headache, nummular headache, hypnic headache or new daily persistent headache (NDPH), preferably selected as migraine.

7. Propofol for use in a method according to one or more of claims 1 to 6 **characterized in that**
a. the human subject responds to propofol in at least one symptom according to claim 4 and/or 5; and
b. the primary headache is selected as migraine; and
c. optionally, the subject does not respond in a symptom of tension-type headache, trigeminal autonomic cephalalgias or other primary headache disorders including primary cough headache, primary exercise headache, primary headache associated with sexual activity, primary thunderclap headache, cold-stimulus headache, external-pressure headache, primary stabbing headache, nummular headache, hypnic headache or new daily persistent headache (NDPH).

8. Propofol for use in a method according to claim 7 **characterized in that** the migraine is selected from the group consisting of migraine without aura, pediatric migraine, menstrual migraine, refractory migraine, chronic migraine, complications of migraine, probable migraine, episodic syndromes associated with migraine, intractable migraine, acute confusional migraine (ACM), migraine with aura including migraine with typical aura, migraine with aura without headache, migraine with brainstem aura, hemiplegic migraine and/or retinal migraine.

9. Propofol for use in a method according to one or more of claims 1 to 8, **characterized in that** the subject post diagnosis is treated for primary headache, in particular with a drug substance selected from the gruop consisting of triptans, ditans such as lasmiditan, NSAIDs, glucocorticoid steroids, salicylates and derivatives thereof, phenylacetic acid and derivatives thereof, 2-phenylpropionic acid and derivatives thereof, 4-aminophenol and derivatives thereof, pyrazolones, selective COX2 inhibitors, anti-depressants, anticonvulsant drugs, opioids, muscle relaxants, barbiturates, benzodiazepines, etomidates, ketamine, propofol, anti-histamines and/or local anesthetics.

10. Propofol for use in a method according to one or more of claims 1 to 9 **characterized in that** a response to propofol is assessed within 240 minutes, 180 minutes, or preferably 120 minutes after administration of propofol to said subject.

11. Propofol for use in a method according to any one or more of claim 1 to 10 **characterized in that** propofol is administered in a subanesthetic dose, preferably in a dose up to 20mg or 2.8mg/kg bdy weigt, respecitvely.

12. Propofol for use in a method according to any one or more of claim 1 to 11 **characterized in that** propofol is administered parenterally, orally, nasally, oculary, aurally, transmucosaly or transdermally, preferably transmucosaly or parenterally.

13. Propofol for use in a method according to one or more of claims 1 to 12 **characterized in that** propofol is administered as single dose.

14. Propofol for use in a method according to one or more of claim 1 to 13 **characterized in that** propofol is administered via a mucoadhesive oral film.

15. Propofol for use in a method of diagnosis of primary headache in a human subject, wherein the responsiveness of the subject to propofol indicates the primary headache.
